(19) Europäisches Patentamt
European Patent Office
Office européen des brevets

(11) Veröffentlichungsnummer: **0 137 169**
**A2**

(12) # EUROPÄISCHE PATENTANMELDUNG

(21) Anmeldenummer: **84109022.8**

(22) Anmeldetag: **31.07.84**

(51) Int. Cl.[4]: **C 07 D 309/38**
A 01 N 43/16, C 07 D 407/12

(30) Priorität: **10.08.83 JP 145158/83**

(43) Veröffentlichungstag der Anmeldung:
**17.04.85 Patentblatt 85/16**

(84) Benannte Vertragsstaaten:
**AT BE CH DE FR GB IT LI NL**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Shiokawa, Kozo**
**210-6, Shukugawara Tama-ku**
**Kawasaki-shi Kanagawa-ken(JP)**

(72) Erfinder: **Kagabu, Shinzo**
**432-131-105, Terada-Machi**
**Hachioji-shi Tokyo(JP)**

(72) Erfinder: **Sakawa, Shinji**
**1-14-13, Tamadaira**
**Hino-shi Tokyo(JP)**

(72) Erfinder: **Matsumoto, Noboru**
**39-15, Namiki-cho**
**Hachioji-shi Tokyo(JP)**

(74) Vertreter: **Schumacher, Günter, Dr. et al,**
**c/o Bayer AG Konzernverwaltung RP Patentabteilung**
**D-5090 Leverkusen 1 Bayerwerk(DE)**

(54) **Cumalinsäureester, Verfahren zu ihrer Herstellung und landwirtschaftliche Fungizide.**

(57) Cumalinsäureester der allgemeinen Formel (I)

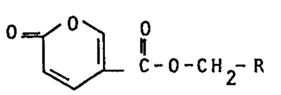

(I)

in welcher R die in der Beschreibung gegebene Bedeutung hat; sowie ihre Verwendung als Fungizide. Die neuen Cumalinsäureester können hergestellt werden, wenn man geeignete Cumalinsäuren oder Cumalinsäurehalogenide mit geeigneten Alkoholen umsetzt.

## Cumalinsäureester, Verfahren zu ihrer Herstellung und landwirtschaftliche Fungizide

Die vorliegende Erfindung betrifft neue Cumalinsäureester, Verfahren zu ihrer Herstellung und ihre Verwendung als Fungizide in Landwirtschaft und Gartenbau.

Es ist bereits bekannt, daß bestimmte Pyranon-Derivate, wie beispielsweise 2,3-H-2,6-Dimethyl-4-keto-5-ethoxycarbonyloxypyran, fungizide Eigenschaften besitzen (vgl. die JP-OS 102 504/1980). Weiterhin ist bekannt, daß Phosphorothiolate, wie beispielsweise S-Benzyl-diisopropylphosphorothiolat, fungizide Eigenschaften aufweisen (vgl. K.H. Büchel, "Pflanzenschutz und Schädlingsbekämpfung", S. 127, Georg Thieme Verlag, Stuttgart 1977). Weiterhin ist bekannt, daß Tetrachloroisophthalsäurenitril ebenfalls fungizide Aktivität besitzt (vgl. die US-PSen 3 290 343 und 3 331 735). Jedoch ist die Wirkung dieser Verbindungen nicht immer zufriedenstellend, insbesondere dann, wenn nur geringe Mengen und Konzentrationen eingesetzt werden.

Die vorliegende Erfindung betrifft speziell Cumalinsäureester der allgemeinen Formel

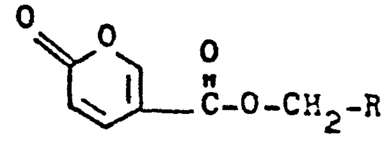

(I)

,

in der

R für eine Cycloalkyl-Gruppe mit 3 bis 8 Kohlenstoff-Atomen, die substituiert sein kann durch wenigstens einen Substituenten ausgewählt aus der aus Niederalkyl-Gruppen, Halogen-Atomen, Phenyl-Gruppen und Halogeno-niederalkenyl-Gruppen bestehenden Klasse, eine Aryl-Gruppe, die substituiert sein kann durch wenigstens einen Substituenten ausgewählt aus der aus Niederalkyl-Gruppen, Halogen-Atomen, Nitro-Gruppen, Phenyl-Gruppen, Methylendioxy-Gruppen und Niederalkoxy-Gruppen bestehenden Klasse, eine Cycloalkenyl-Gruppe mit 5 bis 8 Kohlenstoff-Atomen, die substituiert sein kann durch wenigstens einen Substituenten ausgewählt aus der aus Niederalkenyl-Gruppen und Halogen-Atomen bestehenden Klasse, oder eine verbrückte cyclische Kohlenwasserstoff-Gruppe steht.

Weiterhin wurde gefunden, daß die Cumalinsäureester der allgemeinen Formel (I) erhalten werden mittels eines Verfahrens, in dem ein Cumalinsäure-Derivat der allgemeinen Formel (II)

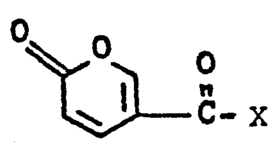

(II) ,

in der

X für ein Halogen-Atom oder Hydroxy steht,

mit einem Alkohol der allgemeinen Formel (III)

$R-CH_2OH$ (III),

in der R die im Vorstehenden angegebene Bedeutung hat, umgesetzt wird.

Die vorliegende Erfindung betrifft ferner Fungizide für Landwirtschaft und Gartenbau, die als Wirkstoff einen Cumalinsäureester der vorstehenden allgemeinen Formel (I) enthalten.

Seitens der Anmelderin wurden theoretische und experimentelle Arbeiten mit dem Ziel der Synthese biologisch aktiver Verbindungen mit neuartigen chemischen Strukturen durchgeführt. Hierbei gelang die Synthese der bisher in der Literatur nicht beschriebenen Cumalinsäureester der allgemeinen Formel (I), und es wurde gefunden, daß diese Verbindungen eine herausragende fungizide Aktivität bei einem Einsatz in Landwirtschaft und Gartenbau besitzen.

Die Untersuchungen der Anmelderin haben ergeben, daß die neuen Verbindungen der allgemeinen Formel (I) leicht mittels allgemeiner Verfahren hergestellt werden können, und daß sie ausgezeichnete Wirkungen als Fungizide für Landwirtschaft und Gartenbau hervorzubringen vermögen, insbesondere gegen die Brusone-Krankheit bei Reis (Pyricularia oryzae), Braunfäule der Tomate (Phytophthora infestans) und Brennfleckenkrankheit der Gurke (Colletotrichum lagenarium).

Die kennzeichnende Eigenschaft der Verbindungen der Formel (I) gemäß der vorliegenden Erfindung besteht darin, daß sie, wie aus der Formel (I) hervorgeht, Cumalinsäure, d.h. α-Pyron-5-carbonsäure als Grundstruktur und die Gruppe $-CH_2-R$ über eine Ester-Bindung

an die Carbonsäure gebunden enthalten. Die herausragende, für Landwirtschaft und Gartenbau nutzbare fungizide Aktivität ist als eine physiologische Aktivität der Verbindungen der Formel (I) hervorzuheben, die durch die angegebene spezielle Struktur bedingt wird.

Die Verbindungen der vorliegenden Erfindung besitzen eine niedrige Toxizität gegenüber warmblütigen Tieren und zeigen in den üblichen Konzentrationen überhaupt keine Phytotoxizität gegen Kulturpflanzen. Infolgedessen bieten sie in bezug auf die Sicherheit ihres Einsatzes Vorteile.

Demgemäß ist es ein Ziel der vorliegenden Erfindung, neue Cumalinsäureester der Formel (I), ein Verfahren für ihre Herstellung sowie ihre Verwendung als Fungizide in Landwirtschaft und Gartenbau verfügbar zu machen.

Diese und andere Ziele der vorliegenden Erfindung werden in der folgenden Beschreibung deutlicher dargestellt.

Die Verbindungen gemäß der vorliegenden Erfindung besitzen ein breites fungizides Spektrum und können in wirksamer Weise gegen verschiedene Pflanzenkrankheiten eingesetzt werden, die beispielsweise durch Archimycetes, Phycomycetes, Ascomycetes, Basidiomycetes und Fungi imperfecti sowie durch verschiedenartige Bakterien verursacht werden.

Die Cumalinsäureester der allgemeinen Formel (I) gemäß der vorliegenden Erfindung können in einfacher Weise beispielsweise mittels des folgenden Verfahrens hergestellt werden:

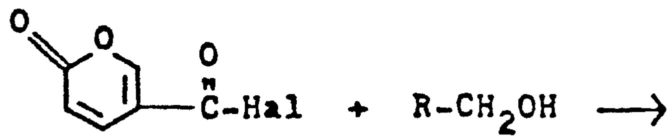

(II) (III)

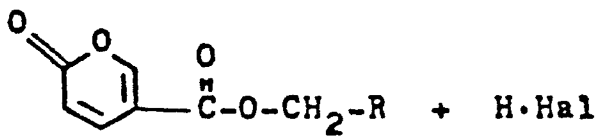

(I)

In dem Formelschema haben R und Hal die im Vorstehenden angegebenen Bedeutungen. Spezielle Beispiele für R sind Cycloalkyl-Gruppen mit 3 bis 8 Kohlenstoff-Atomen, die substituiert sein können, wie Cyclopropyl, Cyclobutyl, Cyclopentyl, Cyclohexyl, Cycloheptyl oder Cyclooctyl. Zu speziellen Beispielen für die Substituenten an den Cycloalkyl-Gruppen zählen Niederalkyl-Gruppen wie Methyl, Ethyl, Propyl, Isopropyl, n- (iso-, sec- oder tert-) Butyl, Halogen-Atome wie Fluor, Chlor, Brom und Iod, die Phenyl-Gruppe und niedere Alkenyl-Gruppen wie Vinyl, Allyl, 1-Propenyl und 1-Butenyl, die durch die gleichen Halogen-Atome, die im Vorstehenden beispielhaft genannt sind, substituiert sein können. Gewählt werden kann wenigstens ein solcher Substituent.

Eine Aryl-Gruppe, die einen Substituenten tragen kann, etwa eine Phenyl- oder α- (oder ß-)-Naphthyl-Gruppe ist ebenfalls ein Beispiel für R. Ein spezielles Beispiel für Substituenten ist wenigstens ein Substituent, der

ausgewählt ist aus der aus den gleichen Niederalkyl-Gruppen und Halogen-Atomen, wie sie oben beispielhaft angegeben wurden, Nitro-Gruppen, Phenyl-Gruppen, Methylendioxy-Gruppen und Niederalkoxy-Gruppen mit den gleichen Niederalkyl-Gruppen wie oben beispielhaft angegeben bestehenden Klasse.

Ein weiteres Beispiel für R ist eine Cycloalkenyl-Gruppe mit 5 bis 8 Kohlenstoff-Atomen, die einen Substituenten tragen kann, beispielsweise eine 1- (oder 2- oder 3-) Cyclopenten-1-yl-, 1- (oder 2- oder 3-) Cyclohexen-1-yl-, 1- (oder 2- oder 3- oder 4-) Cyclohepten-1-yl- oder 1- (oder 2- oder 3- oder 4-) Cycloocten-1-yl-Gruppe. Ein spezielles Beispiel für den Substituenten ist wenigstens ein Substituent, der ausgewählt ist aus der aus den gleichen Niederalkenyl-Gruppen und Halogen-Atomen, wie sie oben beispielhaft angegeben wurden, bestehenden Klasse. Beispiele für die verbrückte cyclische Kohlenwasserstoff-Gruppe, für die R stehen kann, sind Bicyclo/¯2.2.1_7-5-hepten-2-yl-, Adamantyl- und Bicyclo/¯3.1.1_7-hept-7,7-dimethyl-2-yl-Gruppen.

Spezielle Beispiele für Hal sind die gleichen Halogen-Atome, wie sie oben beispielhaft aufgeführt sind, insbesondere Chlor und Brom.

In dem durch das obige Reaktionsschema veranschaulichte Verfahren zur Herstellung der Cumalinsäureester der allgemeinen Formel (I) sind spezielle Beispiele für das Cumalinsäure-Halogenid der allgemeinen Formel (II), in der X Halogen ist, als Ausgangsstoff Cumalylchlorid und Cumalylbromid.

Zu speziellen Beispielen für den Alkohol der allgemeinen Formel (III), der gleichermaßen ein Ausgangsstoff ist, zählen
2,2-Dichloro-3,3-dimethylcyclopropylmethanol,
2,2-Dibromo-3,3-dimethylcyclopropylmethanol,
2,2-Dichloro-1-methylcyclopropylmethanol,
2,2-Dichloro-3-(2,2-dichlorovinyl)cyclopropylmethanol,
1-Phenylcyclopropylmethanol,
2,2-Dichloro-cis-1,3-dimethylcyclopropylmethanol,
2,2-Dichloro-1,3,3-trimethylcyclopropylmethanol,
2,2-Dichloro-3-methylcyclopropylmethanol,
2-Methylbenzylalkohol,
4-Methylbenzylalkohol,
4-Isopropylbenzylalkohol,
2-Bromobenzylalkohol,
4-Bromobenzylalkohol,
2-Nitrobenzylalkohol,
4-Nitrobenzylalkohol,
4-Phenylbenzylalkohol,
2,6-Dichlorobenzylalkohol,
2,6-Dimethylbenzylalkohol,
3,4-Dichlorobenzylalkohol,
2,4-Dichlorobenzylalkohol,
3,5-Dichlorobenzylalkohol,
Piperonylalkohol,
5-Chloro-2-methoxybenzylalkohol,
2,4,5-Trichlorobenzylalkohol,
2,4,6-Trimethylbenzylalkohol,
Perfluorobenzylalkohol,
α-Naphthylmethanol,
Cyclopentylmethanol,
Cyclohexylmethanol,
Cycloheptylmethanol,
1-Cyclohexenylmethanol,
3-Cyclohexenylmethanol,

Perylylalkohol,
5-Norbornen-2-ylmethanol,
Myrtenol,
1-Adamantylmethanol,
2-Chloro-1-cyclohexenylmethanol und
2-Chloro-1-cycloheptenylmethanol.

Das obige Verfahren wird durch das folgende Beispiel speziell veranschaulicht:

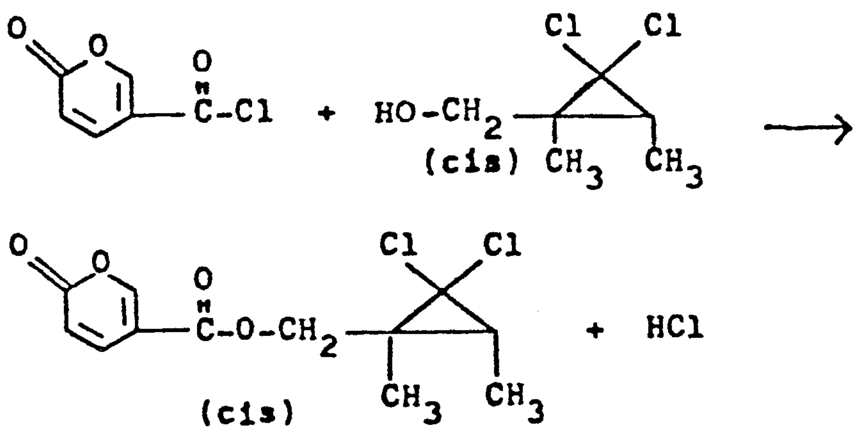

Das vorstehende Verfahren kann zweckmäßigerweise unter Verwendung eines Lösungsmittels oder Verdünnungsmittels durchgeführt werden. Zu diesem Zweck können sämtliche inerten Lösungsmittel und Verdünnungsmittel verwendet werden.

Beispiele für solche Lösungsmittel oder Verdünnungsmittel umfassen aliphatische, cycloaliphatische und aromatische Kohlenwasserstoffe (gegebenenfalls chlorierte) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und

Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Das Verfahren gemäß der vorliegenden Erfindung kann innerhalb eines weiten Temperatur-Bereichs durchgeführt werden. Im allgemeinen kann es bei einer Temperatur zwischen etwa -20°C und dem Siedepunkt der Mischung, günstigerweise zwischen etwa 0°C und etwa 100°C, durchgeführt werden. Zweckmäßigerweise wird die Reaktion unter atmosphärischem Druck durchgeführt, jedoch ist es möglich, unter erhöhtem oder vermindertem Druck zu arbeiten.

Die Cumalinsäureester der allgemeinen Formel können beispielsweise in dem Fall, in dem in der Formel (II) X für Hydroxy steht, auch mittels des folgenden Verfahrens hergestellt werden:

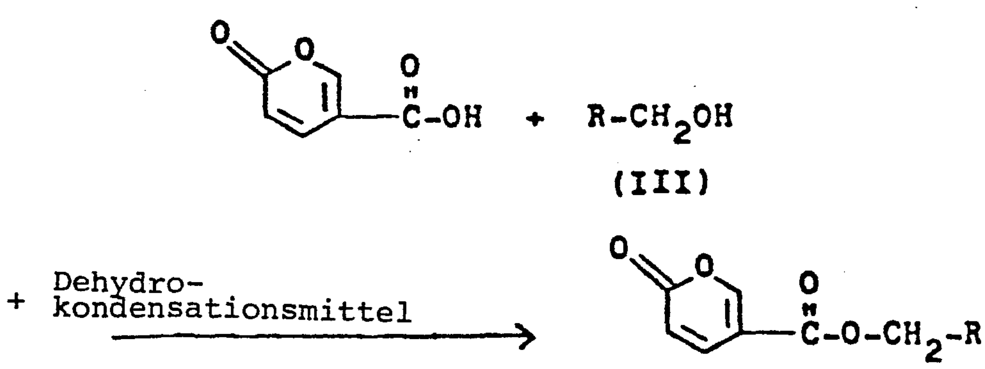


(III)

(I)

In dem Formelschema hat R die im Vorstehenden angegebene Bedeutung. Spezielle Beispiele für R sind diejenigen, die oben bereits beispielhaft genannt wurden.

In dem durch das obige Formelschema dargestellten Verfahren zur Herstellung der Cumalinsäureester der allgemeinen Formel (I) können spezielle Beispiele für den Alkohol der allgemeinen Formel (III) als Ausgangsmaterial die gleichen sein, wie sie bereits oben beispielhaft genannt wurden.

Das oben geschilderte Verfahren wird durch das folgende typische Beispiel speziell veranschaulicht:

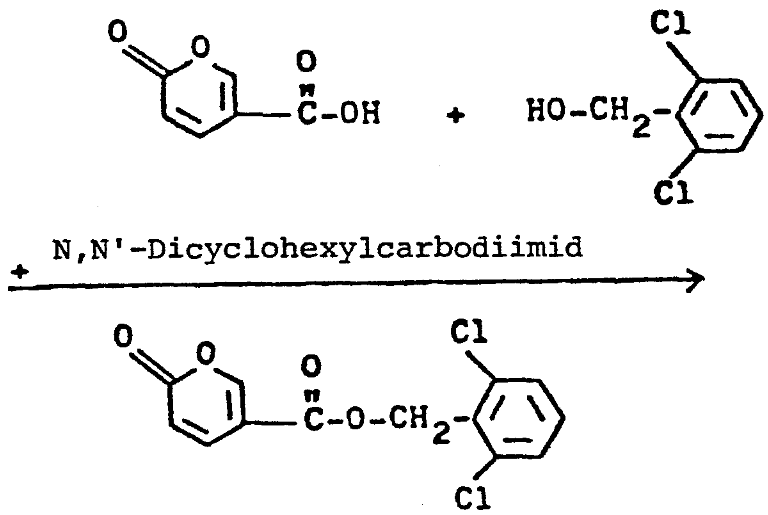


Zur Durchführung des obigen Verfahrens werden zweckmäßigerweise die gleichen inerten Lösungsmittel oder Verdünnungsmittel wie oben angegeben verwendet, und die als Endprodukt angestrebte Verbindung kann gewonnen werden.

Das Verfahren zur Herstellung der aktiven Verbindungen (I) gemäß der vorliegenden Erfindung, in dem in der

Formel (II) X für Hydroxy steht, kann innerhalb eines weiten Temperatur-Bereichs durchgeführt werden. Im allgemeinen kann es bei einer Temperatur zwischen etwa -20°C und dem Siedepunkt der Mischung, günstigerweise zwischen etwa 0°C und etwa 100°C, durchgeführt werden. Zweckmäßigerweise wird die Reaktion unter atmosphärischem Druck durchgeführt, jedoch ist es möglich, unter erhöhtem oder vermindertem Druck zu arbeiten.

Vorzugsweise wird die Reaktion in Gegenwart eines Dehydrokondensationsmittels durchgeführt, und bei dieser Verfahrensweise kann das Endprodukt in hoher Reinheit und mit hoher Ausbeute erhalten werden. N,N'-Dicyclohexylcarbodiimid ist ein spezielles Beispiel für ein solches Dehydrokondensationsmittel.

Zur Anwendung als landwirtschaftliche Fungizide können die Verbindungen (I) gemäß der vorliegenden Erfindung unmittelbar nach Verdünnen mit Wasser oder aber in Form verschiedener Formulierungen eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Einsatz werden diese Präparate entweder unmittelbar oder nach dem Verdünnen mit Wasser auf die gewünschte Konzentration zur Anwendung gebracht. Beispiele für die landwirtschaftlich unbedenklichen Hilfsstoffe, auf die hier Bezug genommen wird, umfassen Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Als Lösungsmittel können Wasser und organische Lösungsmittel verwendet werden. Beispiele für die organischen Lösungsmittel umfassen Kohlenwasserstoffe /¯z.B. n-Hexan, Petrolether, Naphtha, Erdöl-Fraktionen (z.B. Paraffinwachse, Kerosin, Leichtöle, Mittelöle, Schweröle), Benzol, Toluol und Xylole_7, halogenierte Kohlenwasserstoffe (z.B. Methylenchlorid, Kohlenstofftetrachlorid, Trichloroethylen, Ethylenchlorid, Ethylendibromid, Chlorbenzol und Chloroform), Alkohole (z.B. Methylalkohol, Ethylalkohol, Propylalkohol und Ethylenglycol), Ether (z.B. Diethylether, Ethylenoxid und Dioxan), Alkohol-ether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anionische oberflächenaktive Mittel wie Alkylschwefelsäureester (z.B. Natriumlaurylsulfat), Arylsulfonsäuren

(z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z.B. Trichlorofluoromethan, Dichlorofluoromethan, 1,2,2-Trichloro-1,1,2-trifluoroethan, Chlorbenzol, verflüssigtes Erdgas und niedere Ether), die Verbrennung steuernde Mittel für Räucherpräparate (z.B. Nitrite, Zink-Pulver und Dicyandiamid), Sauerstoff abgebende Mittel (z.B. Chlorate), wirkungsverlängernde Mittel, Dispersions-Stabilisatoren /¯z.B. Casein, Tragant, Carboxymethylcellulose (CMC) und Polyvinylalkohol (PVA)_7 und synergistische Mittel.

Die Verbindungen gemäß der vorliegenden Erfindung können mittels allgemein bei der Herstellung von Agrochemikalien angewandter Methoden zu verschiedenartigen Präparaten formuliert werden. Erläuternde Beispiele für solche Anwendungsformen sind emulgierbare Konzentrate,

Öl-Präparate, benetzbare Pulver, lösliche Pulver, Suspensionen, Stäubemittel, Granulat, Pulverpräparate, Räuchermittel, Tabletten, Aerosole, Pasten und Kapseln.

Die land- und gartenwirtschaftlichen Fungizide gemäß der vorliegenden Erfindung können etwa 0,1 bis etwa 95 Gew.-%, vorzugsweise etwa 0,5 bis etwa 90 Gew.-% des vorerwähnten aktiven Wirkstoffes enthalten.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,0001 bis etwa 20 Gew.-%, vorzugsweise etwa 0,005 bis etwa 10 Gew.-%.

Der Gehalt des Wirkstoffs kann in geeigneter Weise je nach der Form des Präparats oder Mittels, dem Verfahren, Zweck, der Zeit und dem Ort seiner Anwendung, dem Zustand des Auftretens einer Pflanzenkrankheit etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, anderen Fungiziden, Mitiziden, Nematiziden, Anti-Virus-Mitteln, selektiven Herbiziden, Pflanzen-Wachstumsregulatoren und Lockstoffen /̲ Organophosphat-Verbindungen, Carbamat-Verbindungen, Dithio-(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen_7 und/oder Düngemitteln.

Die den im Vorstehenden bezeichneten Wirkstoff enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.), Räuchern, Bodenbehandlung (Vermischen mit dem Boden, Streuen, Bedampfen, Gießen etc.), Oberflächenbehandlung (Beschichten, Bändern, Bestäuben, Bedecken etc.) und Eintauchen. Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmengen pro Flächeneinheit betragen beispielsweise etwa 0,03 bis etwa 10 kg/ha, vorzugsweise etwa 0,3 bis etwa 6 kg/ha. In besonders gelagerten Fällen können oder sollten sogar die Aufwandmengen außerhalb des angegebenen Bereichs liegen.

Gemäß der vorliegenden Erfindung kann ein fungizides Mittel für die Landwirtschaft verfügbar gemacht werden, das eine Verbindung der allgemeinen Formel (I) als Wirkstoff und ein Verdünnungsmittel (ein Lösungsmittel und/oder ein Streckmittel und/oder einen Träger) und/oder ein oberflächenaktives Mittel und, sofern weiter erforderlich, einen Stabilisator, ein Haftmittel, ein synergistisches Mittel etc. enthält.

Die vorliegende Erfindung stellt ebenfalls ein Verfahren zur Bekämpfung von Erkrankungen von Nutzpflanzen

zur Verfügung, bei dem auf pathogene Pilze und/oder deren Lebensraum und den Ort des Auftretens der Erkrankungen eine Verbindung der Formel (I) allein oder im Gemisch mit einem Verdünnungsmittel (einem Lösungsmittel und/oder einem Streckmittel und/oder einem Träger) und/oder einem oberflächenaktiven Mittel und, sofern weiter erforderlich, einem Stabilisator, einem Haftmittel, einem synergistischen Mittel etc. aufgebracht wird.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Beispiel 1 (Synthesebeispiel)

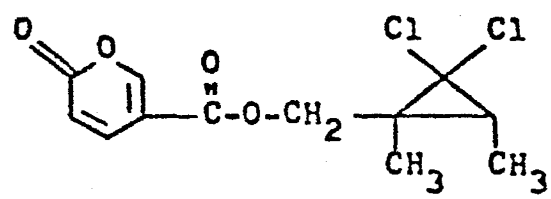

(Verbindung Nr. 1)

20 ml 2,2-Dichloro-cis-1,3-dimethyl-cyclopropylmethanol wurden in 30 ml Toluol gelöst, und die Lösung wurde allmählich zu 10 g Cumalylchlorid hinzugefügt. Die Mischung wurde langsam auf 100°C erhitzt und 5 h bei dieser Temperatur gerührt. Dann wurde die Temperatur auf Raumtemperatur gesenkt, und 50 ml n-Hexan wurden zugesetzt. Die Lösung wurde 12 h stehen gelassen, und

die ausgefallenen Kristalle wurden durch Filtration gesammelt und aus n-Hexan umkristallisiert, wonach 7,8 g des gesuchten 2,2-Dichloro-cis-1,3-dimethylcyclopropylmethylcumalats mit dem Schmp. 105-106°C erhalten wurden.

Beispiel 2 (Synthesebeispiel)

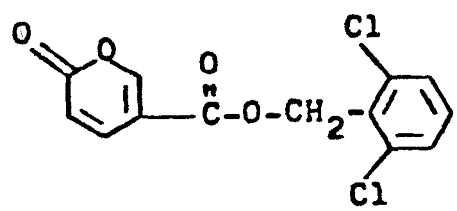

(Verbindung Nr. 2)

Cumalinsäure (2,8 g) und 3,54 g 2,6-Dichlorobenzylalkohol wurden in 50 ml Tetrahydrofuran gelöst, und unter Rühren wurden 3,8 g Dicyclohexylcarbodiimid und anschließend 100 mg 4-Dimethylaminopyridin zugegeben. Das Rühren wurde fortgesetzt. 5 h später wurden 50 ml Toluol hinzugefügt, und die Mischung wurde weitere 5 h gerührt. Die unlöslichen Stoffe wurden durch Filtration entfernt, und das Filtrat wurde eingeengt. Toluol (100 ml) wurde zu dem Konzentrat hinzugefügt, und die Mischung wurde 1 h mit Eiswasser gekühlt. Die unlöslichen Stoffe wurden durch Filtration entfernt. Das Filtrat wurde nacheinander mit einer 1-proz. wäßrigen Salzsäure-Lösung, mit einer 1-proz. wäßrigen Natriumhydrogencarbonat-Lösung und mit Wasser gewaschen und dann über Natriumsulfat getrocknet. Das Trockenmittel wurde durch Filtration entfernt, und das Toluol wurde

unter vermindertem Druck abgedampft. Die abgeschiedenen Kristalle wurden in heißem Ethanol gelöst, und die Lösung wurde durch Aktivkohle hindurchgeleitet. Das Ethanol wurde auf ein Volumen von etwa 20 ml eingeengt, und die Lösung wurde bei Raumtemperatur stehen gelassen, wonach 4,86 g des gesuchten 2,6-Dichlorobenzylcumalats in Form farbloser, nadelartiger Kristalle mit dem Schmp. 104-105°C erhalten wurden.

Nach im wesentlichen dem gleichen Verfahren wie in Beispiel 1 wurden die in der nachfolgenden Tabelle 1 aufgeführten Verbindungen Nr. 3, 4, 6, 7, 10, 11, 14, 15, 17, 18, 19, 20, 22, 23, 24, 26, 28, 31, 34 und 38 synthetisiert. Weiterhin wurden nach im wesentlichen dem gleichen Verfahren wie in Beispiel 2 die in der nachfolgenden Tabelle 1 aufgeführten Verbindungen Nr. 5, 8, 12, 13, 16, 21, 25, 27, 30, 32, 33, 34, 35, 36 und 37 synthetisiert.

Tabelle 1

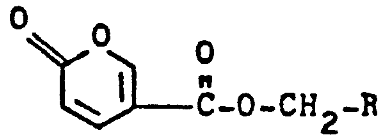

| Verbindung Nr. | R | Physikal. Konstante (Schmp., °C) |
|---|---|---|
| 3 | 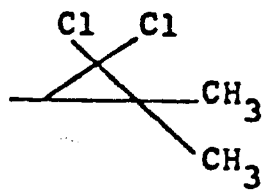 | 127.5 - 128 |
| 4 | 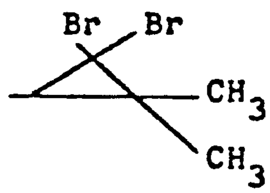 | 124 |
| 5 | 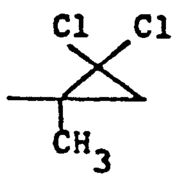 | 99 - 101 |
| 6 | 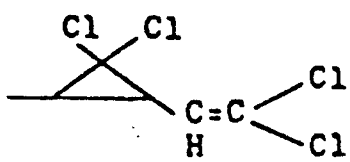 | 106 - 108 |

Tabelle 1 - Fortsetzung

| Verbindung Nr. | R | Physikal. Konstante (Schmp., °C) |
| --- | --- | --- |
| 7 | 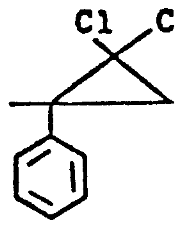 | 78 - 80 |
| 8 | 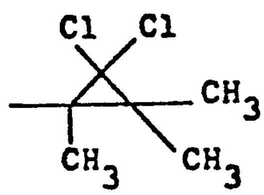 | 122.5 - 124 |
| 9 | 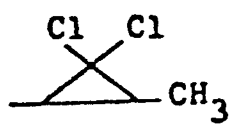 | 72 |
| 10 | 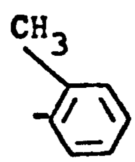 | 97 - 99 |
| 11 | 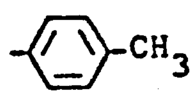 | 104 |
| 12 | 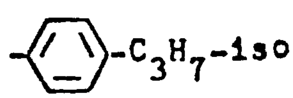 | 150 |
| 13 | 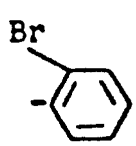 | 97 |
| 14 | 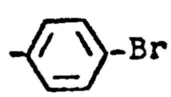 | 96 |
| 15 | 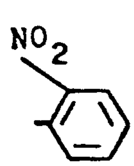 | 113 |

Nit 165

Tabelle 1 - Fortsetzung

| Verbindung Nr. | R | Physikal. Konstante (Schmp., °C) |
| --- | --- | --- |
| 16 | 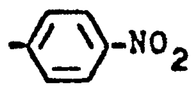 | 151 |
| 17 | 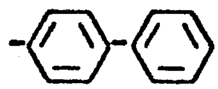 | 104 - 106 |
| 18 | 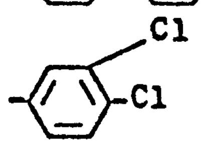 | 115 - 116 |
| 19 | 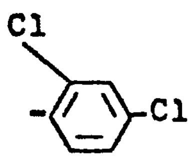 | 100 - 102 |
| 20 | 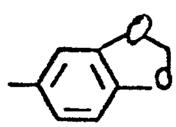 | 85 - 87 |
| 21 | 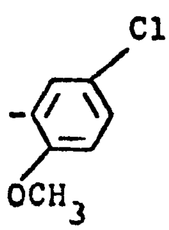 | 85 - 87 |
| 22 | 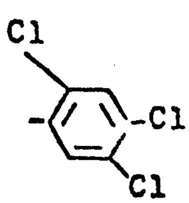 | 132 |
| 23 | 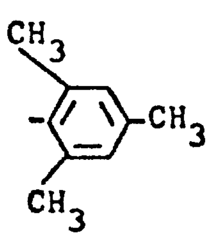 | 102 - 104 |

Tabelle 1 - Fortsetzung

| Verbindung Nr. | R | Physikal. Konstante (Schmp., °C) |
|---|---|---|
| 24 | 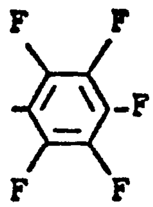 | 88 - 89 |
| 25 | 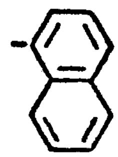 | 107 - 108 |
| 26 | 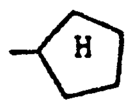 | 73.5 - 75 |
| 27 | 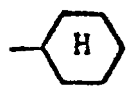 | 70 - 71.5 |
| 28 | 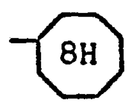 | 57 - 59.5 |
| 29 | 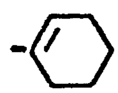 | 74 - 76 |
| 30 | 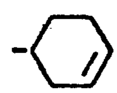 | 80 - 81 |
| 31 | 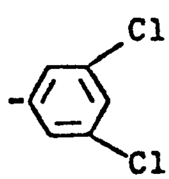 | 107 - 108 |
| 32 | 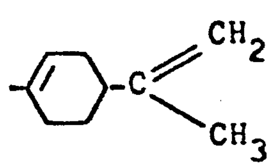 | 97 - 98 |

Tabelle 1 - Fortsetzung

| Verbindung Nr. | R | Physikal. Konstante (Schmp., °C) |
|---|---|---|
| 33 | 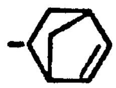 | 92 - 94 |
| 34 | 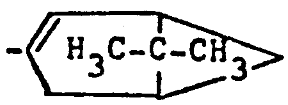 | 56 - 57 |
| 35 | 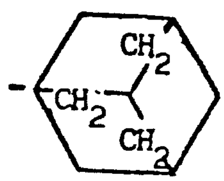 | 108 |
| 36 | 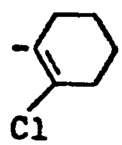 | 73 - 74 |
| 37 | 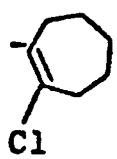 | 97 - 98 |
| 38 | 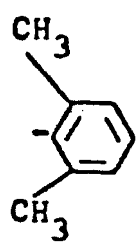 | 124 - 126 |

Beispiel 3 (Benetzbares Pulver)

15 Teile der Verbindung Nr. 1 der Erfindung, 80 Teile eines Gemisches (1:5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf einen pathogenen Pilz und/oder seinen Lebensraum und den Ort, an dem eine Pflanzenkrankheit auftritt, aufgesprüht.

Beispiel 4 (Emulgierbares Konzentrat)

30 Teile der Verbindung Nr. 2 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf einen pathogenen Pilz und/oder seinen Lebensraum und den Ort, an dem eine Pflanzenkrankheit auftritt, aufgesprüht.

Beispiel 5 (Stäubemittel)

2 Teile der Verbindung Nr. 3 der Erfindung und 98 Teile Tonpulver werden pulverisiert und gemischt, wodurch ein Stäubemittel hergestellt wird. Dieses wird über einem pathogenen Pilz und/oder seinen Lebensraum und den Ort, an dem eine Pflanzenkrankheit auftritt, ausgestreut.

Beispiel 6 (Stäubemittel)

Die Verbindung Nr. 34 der Erfindung (1,5 Teile), 0,5 Teile Isopropylhydrogenphosphat (PAP) und 98 Teile Tonpulver werden pulverisiert und gemischt, wodurch ein Stäubemittel hergestellt wird. Dieses wird über einem pathogenen Pilz und/oder seinem Lebensraum und dem Ort, an dem eine Pflanzenkrankheit auftritt, ausgestreut.

Beispiel 7 (Granulat)

Wasser (25 Teile) wird zu einer Mischung aus 10 Teilen der Verbindung Nr. 3 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat hergestellt wird. Das Granulat wird über einem pathogenen Pilz und/oder seinem Lebensraum und dem Ort, an dem eine Pflanzenkrankheit auftritt, ausgestreut.

Beispiel 8 (Biologischer Test)

Test auf Bekämpfung der Brennfleckenkrankheit der Gurke:

Test-Verfahren:

Eine Test-Verbindung in Form einer Emulsion, die gemäß Beispiel 4 hergestellt worden war, wurde auf in unglasierten 9 cm-Töpfen gezogene Gurken (Varietät: "Suyo") im Zweiblatt-Stadium mit einer Sprühpistole aufgesprüht. Einen Tag nach dem Sprühen wurden die Pflanzen

mit einer Sporen-Suspension von Colletotrichum lagenarium durch Sprühen inokuliert, und die Pflanzen wurden über Nacht in einem Raum mit einer konstanten Temperatur von 23°C und einer Luftfeuchtigkeit von wenigstens 90 % stehen gelassen. Sechs Tage später wurde der Grad der Erkrankung durch das Verhältnis der Läsionen zeigenden Flächen bestimmt, und der Bekämpfungsindex wurde berechnet.

$$\text{Bekämpfungs-Index (\%)} = 100 - \frac{\text{Grad der Erkrankung der behandelten Fläche}}{\text{Grad der Erkrankung der unbehandelten Fläche}} \times 100$$

Die Ergebnisse sind in der Tabelle 2 aufgeführt.

Tabelle 2

| Verbindung Nr. | Wirkstoff-Konzentration (ppm) | Bekämpfungs-Index (%) |
|---|---|---|
| 1 | 500 | 100 |
| 2 | 500 | 100 |
| 3 | 500 | 100 |
| 4 | 500 | 100 |
| 5 | 500 | 100 |
| 8 | 500 | 100 |
| 9 | 500 | 100 |
| 12 | 500 | 100 |
| 28 | 500 | 100 |
| 30 | 500 | 100 |
| 32 | 500 | 100 |
| 34 | 500 | 100 |
| 35 | 500 | 100 |
| 37 | 500 | 100 |
| Vergleich | | |
| A-1 | 1000 | 80 |
| TPN (Handelsprodukt) | 500 | 95 |

Anmerkung: Vergleichsverbindungen

A-1

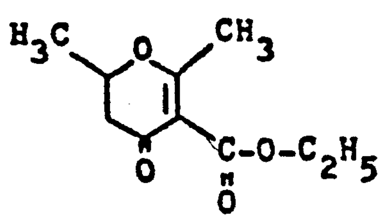

(die in der JP-OS 102504/1980 beschriebene Verbindung)

TPN: Tetrachloroisophthalsäurenitril (75-proz. benetzbares Pulver).

Nit 165

Beispiel 9 (Biologischer Test)

Test auf Bekämpfung der Braunfäule der Tomate:

Test-Verfahren:

Eine Test-Verbindung in Form einer Emulsion, die gemäß Beispiel 4 hergestellt worden war, wurde auf in unglasierten 9 cm-Töpfen gezogene Tomaten (Varietät: "Kurihara") im Zwei- bis Dreiblatt-Stadium mit einer Sprühpistole aufgesprüht. Einen Tag nach dem Sprühen wurden die Pflanzen mit einer Sporen-Suspension von Phytophthora infestans durch Sprühen inokuliert, und die Pflanzen wurden über Nacht in einem Raum mit einer konstanten Temperatur von 22°C und einer Luftfeuchtigkeit von wenigstens 90 % stehen gelassen. Fünf Tage später wurde der Grad der Erkrankung durch das Verhältnis der Läsionen zeigenden Flächen bestimmt, und der Bekämpfungsindex wurde in der gleichen Weise wie in Beispiel 8 berechnet. Die Ergebnisse sind in der Tabelle 3 aufgeführt.

Tabelle 3

| Verbindung Nr. | Wirkstoff-Konzentration (ppm) | Bekämpfungs-Index (%) |
|---|---|---|
| 1 | 500 | 100 |
| 2 | 500 | 100 |
| 3 | 500 | 100 |
| 4 | 500 | 100 |
| 5 | 500 | 100 |
| 6 | 500 | 100 |
| 10 | 500 | 100 |
| 13 | 500 | 100 |
| 14 | 500 | 100 |

Nit 165

Tabelle 3 - Fortsetzung

| Verbindung Nr. | Wirkstoff-Konzentration (ppm) | Bekämpfungs-Index (%) |
|---|---|---|
| 15 | 500 | 100 |
| 17 | 500 | 100 |
| 19 | 500 | 100 |
| 20 | 500 | 100 |
| 21 | 500 | 100 |
| 22 | 500 | 100 |
| 23 | 500 | 100 |
| 24 | 500 | 100 |
| 25 | 500 | 100 |
| 26 | 500 | 100 |
| 27 | 500 | 100 |
| 34 | 500 | 100 |
| 36 | 500 | 100 |
| Vergleich | | |
| TPN (Handelsprodukt) | 500 | 90 |

Anmerkung: Bei TPN handelt es sich um das gleiche Produkt, wie es in der Fußnote zu Tabelle 2 angegeben ist.

Beispiel 10 (Biologischer Test):

Test auf Bekämpfung der Brusone-Krankheit von Reis:
Herstellung einer Test-Verbindung:

Wirkstoff: 50 Gew.-Teile;
Träger: 45 Gew.-Teile einer Mischung (1 : 5) aus Diatomeenerde und Kaolin;
Emulgator: 5 Gew.-Teile Polyoxyethylenalkylphenylether.

Die aktive Verbindung, der Träger und der Emulgator wurden in den angegebenen Mengen miteinander vermischt und pulverisiert, wodurch ein benetzbares Pulver gebildet wurde. Eine vorher festgelegte Menge des benetzbaren Pulvers wurde mit Wasser verdünnt, wodurch ein Test-Präparat gebildet wurde.

Test-Verfahren

Reispflanzen (Varietät: Asahi) wurden in unglasierten Porzellan-Töpfen mit einem Durchmesser von 12 cm kultiviert. Im dritten bis vierten Wachstums-Stadium der Reispflanzen wurde die auf eine vorher festgelegte Konzentration verdünnte Test-Verbindung in einer Menge von 50 ml auf jeweils drei Töpfe aufgesprüht. Am nächsten Tage wurden die Reispflanzen durch zweimaliges Besprühen mit einer künstlich kultivierten Sporensuspension inokuliert. Die Pflanzen wurden bei 25°C und einer relativen Luftfeuchtigkeit von 100 % gehalten, um die Pflanzen zu infizieren. Sieben Tage nach der Beimpfung wurde der Grad der Erkrankung pro Topf aufgrund des folgenden Bewertungsmaßstabs bestimmt, und der Bekämpfungs-Index (%) wurde berechnet.

| Grad der Erkrankung | Verhältnis der Fläche der Verletzungen (%) |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Diese Ergebnisse wurden aus drei Töpfen pro Prüfung erhalten. Die Ergebnisse sind in der nachstehenden Tabelle 4 aufgeführt.

Tabelle 4

| Verbindung Nr. | Wirkstoff-Konzentration (ppm) | Bekämpfungs-Index (%) |
|---|---|---|
| 1 | 500 | 100 |
| 3 | 500 | 100 |
| 7 | 500 | 100 |
| 8 | 500 | 100 |
| 9 | 500 | 100 |
| 12 | 500 | 95 |
| 14 | 500 | 95 |
| 16 | 500 | 90 |
| 18 | 500 | 100 |
| 21 | 500 | 95 |
| 24 | 500 | 100 |
| 25 | 500 | 95 |
| 29 | 500 | 95 |
| 31 | 500 | 95 |
| 33 | 500 | 100 |
| 34 | 500 | 100 |
| 36 | 500 | 100 |
| 37 | 500 | 100 |

Tabelle 4 - Fortsetzung

| Verbindung Nr. | Wirkstoff-Konzentration (ppm) | Bekämpfungs-Index (%) |
|---|---|---|
| Vergleich | | |
| IBP (Handelsprodukt) | 500 | 78 |

Anmerkung: Bei IBP handelt es sich um S-Benzyl-diisopropyl-phosphorothiolat (48-proz. emulgierbares Konzentrat).

Bei den in den Beispielen 8, 9 und 10 durchgeführten Tests wurde keinerlei Phytotoxizität beobachtet.

Patentansprüche

1. Cumalinsäureester der allgemeinen Formel (I)

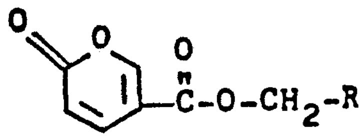

(I)

in der

R für eine Cycloalkyl-Gruppe mit 3 bis 8 Kohlenstoff-Atomen steht, die gegebenenfalls substituiert ist durch wenigstens eine Niederalkyl-Gruppe, Halogen-Atome, Phenyl-Gruppen und Halogeno-niederalkenyl-Gruppen, für eine Aryl-Gruppe, die gegebenenfalls substituiert ist durch wenigstens eine Niederalkyl-Gruppe, Halogen-Atome, Nitro-Gruppen, Phenyl-Gruppen, Methylendioxy-Gruppen und Niederalkoxy-Gruppen, für eine Cycloalkenyl-Gruppe mit 5 bis 8 Kohlenstoff-Atomen, die gegebenenfalls substituiert ist durch wenigstens eine Niederalkenyl-Gruppe und Halogen-Atome, oder für eine verbrückte cyclische Kohlenwasserstoff-Gruppe steht.

2. Cumalinsäureester nach Anspruch 1, wobei in der Formel (I)

R für gegebenenfalls durch Methyl, Ethyl, n- oder iso-Propyl, n-, iso-, sec.- und tert.

Butyl, Fluor, Chlor, Brom, Jod, Phenyl, Halogenphenyl, Alkenyl oder Halogenalkenyl substituiertes Cycloalkyl mit 3 bis 8 Kohlenstoffatomen steht, für gegebenenfalls durch mindestens eine Methyl-, Ethyl-, n- oder iso-Propyl-, n-, iso-, sec.- und tert. Butyl-, Fluor-, Chlor-, Brom-, Jod-, Nitro-, Phenyl-, Methylendioxy-, Methoxy-, Ethoxy-, n- oder iso-Propoxy-, n-, iso-, sec.- oder tert.-Butoxy-Gruppe substituiertes Phenyl oder α- oder -β-Naphthyl steht, für gegebenenfalls durch mindestens eine Methyl-, Ethyl-, n- oder iso-Propyl-, n-, iso-, sec.- und tert.-Butyl-, Fluor-, Chlor-, Brom- oder Jodgruppe substituiertes Cycloalkenyl mit 5 bis 8 Kohlenstoffatomen steht, für Bicyclo-[2.2.1]-5-hepten-yl, Adamantyl oder Bicyclo-[3.1.1]-hept-7,7-dimethyl-2-yl steht.

3. Verfahren zur Herstellung von Cumalinsäureestern der Formel (I)

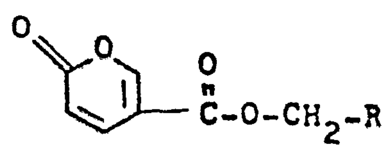

(I)

in der

R für eine Cycloalkyl-Gruppe mit 3 bis 8 Kohlenstoff-Atomen, die gegebenenfalls substituiert ist durch wenigstens eine Niederalkyl-Gruppe, Halogen-Atome, Phenyl-Gruppen und Halogenoniederalkenyl-Gruppen, für eine Aryl-Gruppe, die gegebenenfalls substituiert ist durch wenigstens eine Niederalkyl-Gruppe, Halogen-Atome, Nitro-Gruppen, Phenyl-Gruppen, Methylendioxy-Gruppen und Niederalkoxy-Gruppen, für eine Cycloalkenyl-Gruppe mit 5 bis 8 Kohlenstoff-Atomen, die gegebenenfalls substituiert ist durch wenigstens eine Niederalkenyl-Gruppe und Halogen-Atome, oder für eine verbrückte cyclische Kohlenwasserstoff-Gruppe steht,

dadurch gekennzeichnet, daß ein Cumalinsäure-Derivat der allgemeinen Formel (II)

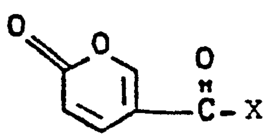

(II)

,

in der

X für ein Halogen-Atom oder Hydroxy steht,

mit einem Alkohol der allgemeinen Formel (III)

$$R-CH_2OH \quad (III),$$

in der R die im Vorstehenden angegebene Bedeutung hat, umgesetzt wird.

4. Mittel zur Bekämpfung von Pilzen, dadurch gekennzeichnet, daß sie wenigstens einen Cumalinsäureester der allgemeinen Formel (I) nach einem der Ansprüche 1 und 3 enthalten.

5. Verwendung von Cumalinsäureestern der allgemeinen Formel (I) nach einem der Ansprüche 1 und 3 zur Bekämpfung von Pilzen in der Landwirtschaft und im Gartenbau.

6. Verfahren zur Bekämpfung von Pilzen, dadurch gekennzeichnet, daß man Cumalinsäureester der allgemeinen Formel (I) nach einem der Ansprüche 1 und 3 auf Pilze und/oder deren Umwelt einwirken läßt.

7. Verfahren zur Herstellung von Mitteln für die Bekämpfung von Pilzen, dadurch gekennzeichnet, daß Cumalinsäureester der allgemeinen Formel (I) nach einem der Ansprüche 1 und 3 mit Streckmitteln und/oder oberflächenaktiven Mitteln vermischt werden.